# EUROPEAN PATENT SPECIFICATION

(11) **EP 2 421 362 B1**
(45) Date of publication and mention of the grant of the patent: **17.05.2017**
(21) Application number: 10718253.7
(22) Date of filing: 23.04.2010
(51) Int. Cl.: A01N 25/30, C07C 43/10, C07C 43/11, C07C 43/15, C11D 1/722, A01N 43/653, A01N 43/90, A01N 41/06, A01N 41/10, A01N 47/36, A01N 45/02, A01N 51/00, A01P 3/00, A01P 13/00, A01P 7/04

(54) **ALCOHOL ALKOXYLATES AS ADJUVANTS FOR AGROCHEMICAL FORMULATIONS**
ALKOHOLALKOXYLATE ALS ADJUVANS FÜR AGROCHEMISCHE FORMULIERUNGEN
ALCOOL ALCOXYLATES COMME ADJUVANTS POUR DES FORMULATIONS AGROCHIMIQUES

(30) Priority: 23.04.2009 GB 0907003
(43) Date of publication of application: 29.02.2012
(60) Divisional application: 17161850.7
(73) Proprietor: Syngenta Limited, Guildford, Surrey GU2 7YH (GB)
(72) Inventor: BELL, Gordon, Alastair, Bracknell RG42 6RY Berkshire (GB)
(74) Representative: Syngenta International AG
(86) International application number: PCT/GB2010/000821
(87) International publication number: WO 2010/122313

(56) References cited:
- EP-A1- 0 652 281
- WO-A1-03/022048
- WO-A1-2008/132150
- WO-A1-2009/088778
- WO-A1-2009/130281
- WO-A2-2005/084435
- WO-A2-2009/130282
- DATABASE WPI Week 200560 Thomson Scientific, London, GB; AN 2005-585477 XP002592674 & JP 2005 200640 A (NIPPON OILS & FATS CO LTD) 28 July 2005 (2005-07-28)
- DATABASE WPI Week 200377 Thomson Scientific, London, GB; AN 2003-820150 XP002592675 & JP 2003 226900 A (NIPPON OILS & FATS CO LTD) 15 August 2003 (2003-08-15)

## Description

### FORMULATION

This invention relates to agrochemical pesticidal formulations comprising bioperformance enhancing adjuvants and to use of such adjuvants.

Numerous adjuvants which enhance the bioperformance of agrochemicals are known. A broad mention of alcohol alkoxylates as penetration aids is made in WO2008/037375 and in WO2005084435. This last document describes alcohol alkoxylates as penetration enhancers for agrochemicals, including butylenoxide/ethylenoxide copolymers but different from the ones of the present invention in the structure of the BO unit. WO 2009/130281, relevant under Art. 54 (3) EPC, discloses alcohol alkoxylate adjuvants for agrochemical formulations. Specific compounds mentioned in that document have been excluded from formula (I) of claim 1. The present invention provides an agrochemical pesticidal formulation as claimed in
claim 1.

Each alkyl chain is, independently, straight or branched.

Optional substituents on the alkyl and alkenyl groups are, independently, hydroxy and epoxy groups.

Certain butylene oxide-ethylene oxide block copolymers are disclosed by J.Chlebicki in J.Colloid and Interface Science 206, 77-82 (1998).

Suitably R₂ is hydrogen or C₁₋₂ alkyl; more suitably R₂ is hydrogen or methyl; most suitably R₂ is hydrogen.

Suitably R₁ is non-substituted alkyl.

BO [butylene oxide] has the empirical formula C₄H₈O. Every BO unit has the formula CH(R₄)CH(R₅)O but each BO unit is independently selected from the following options: R₄ is methyl and R₅ is methyl; or R₄ is ethyl and R₅ is hydrogen; or R₄ is hydrogen and R₅ is ethyl.

Propylene oxide [PO] has the empirical formula C₃H₆O. Every PO unit has the formula CH(R₆)CH(R₇)O but each PO unit is independently selected from the following options: R₆ is methyl and R₇ is hydrogen; or R₆ is hydrogen and R₇ is methyl.

In one aspect, the block [AO]ₘ is a PO block followed by an EO block, where the PO block is bonded to the BO block, such that the adjuvant is of formula (Ib):

R₁O[BO]ₙ[PO]_{m'}[EO]_{m''}R₂ (Ib)

where (m' + m" = m) and R₁, R₂, n and m are as defined for compounds of formula (I).

Suitably AO is ethylene oxide.

Suitably n is from 2 to 8; more suitably from 3 to 6; even more suitably it is 4.

Suitably m is from 8 to 12; more suitably it is 10.

The bioperformance enhancing adjuvants of the present invention may be used effectively at much lower concentrations than the effective concentrations for conventional adjuvants.

The bioperformance enhancing adjuvants of the present invention may be used synergistically with other bioperformance enhancing adjuvants of the present invention or with conventional adjuvants.

The values of n and m represent values both for individual species and for averages taken over a distribution of compounds. This will be well understood by the skilled person.

Suitably the bioperformance enhancing adjuvants of the present invention are used to enhance the bioperformance of a pesticide. Pesticides suitable for use with the present invention include insecticides, fungicides, herbicides, acaricides, nematicides and biocides suitable for controlling pests, diseases or weeds that are a problem in agriculture. Many such pesticides are known and are described in The Pesticide Manual 14th edition published by the British Crop Protection Council in 2006.

The invention is illustrated by the following non-limiting Examples in which all parts and percentages are by weight unless otherwise stated.

### EXAMPLE 1

This example illustrates compounds of the present invention. Twenty eight butylene oxide based compounds were synthesised using standard techniques familiar to a skilled person [for example, see EP0681865A]. Each sample consists of a hydrocarbon tail connected to a butylene oxide section which in turn is connected to an ethylene oxide section; the samples are compounds of formula (I) in which AO is ethylene oxide and R₂ is hydrogen; and R₁, n and m are as defined in Table 1. Samples 1 to 3 and 24 to 27 were prepared using branched alcohols containing an average of 13 carbon atoms. Samples 4 to 7 were prepared with 2-ethylhexyl alcohol. Samples 8 to 11 were prepared using butanol as the starting solvent. Samples 12 to 14 and sample 28 used a fraction of alcohols ranging from 12 to 15 carbons in length. Samples 15 to 23 used an alcohol range from C₁₂ to C₁₆. The values of n and m are average values.

Samples 8, 9, 10, 11, 28, 29 and 30 are not of the present invention.

**Table 1**

| **Sample** | **R₁ alkyl chain** | **n** | **m** |
|---|---|---|---|
| 1 | C₁₃ [average] | 2 | 8 |
| 2 | C₁₃ [average] | 4 | 8 |
| 3 | C₁₃ [average] | 6 | 12 |
| 4 | 2-ethylhexyl | 2 | 6 |
| 5 | 2-ethylhexyl | 2 | 3 |
| 6 | 2-ethylhexyl | 4 | 6 |
| 7 | 2-ethylhexyl | 6 | 10 |
| 8 | C₄ | 2 | 8 |
| 9 | C₄ | 4 | 10 |
| 10 | C₄ | 4 | 8 |
| 11 | C₄ | 6 | 8 |
| 12 | C₁₂₋₁₅ | 2 | 9 |
| 13 | C₁₂₋₁₅ | 4 | 10 |
| 14 | C₁₂₋₁₅ | 6 | 12 |
| 15 | C₁₂₋₁₆ | 1 | 3 |
| 16 | C₁₂₋₁₆ | 1 | 5 |
| 17 | C₁₂₋₁₆ | 1 | 8 |
| 18 | C₁₂₋₁₆ | 2 | 5 |
| 19 | C₁₂₋₁₆ | 2 | 8 |
| 20 | C₁₂₋₁₆ | 3 | 5 |
| 21 | C₁₂₋₁₆ | 3 | 8 |
| 22 | C₁₂₋₁₆ | 4 | 3 |
| 23 | C₁₂₋₁₆ | 4 | 8 |
| 24 | C₁₃ [average] | 2 | 3 |
| 25 | C₁₃ [average] | 2 | 8 |
| 26 | C₁₃ [average] | 4 | 3 |
| 27 | C₁₃ [average] | 4 | 8 |
| 28 | C₁₂₋₁₅ | 4 | 0 |
| 29 | C₁₈ (oleyl) | 4 | 0 |
| 30 | C₁₈ (oleyl) | 4 | 20 |

### EXAMPLE 2

This example shows that samples 15, 17, 24 and 25 from Table 1 behave as adjuvants for fungicides used against brown rust (puccinia recondita). Wheat was grown outside in field plots. Each plot was sprayed with water at a rate of 15 litres per hectare, the water containing either difenoconazole or cyproconazole, at a concentration which enabled a pesticide application rate of 0.1, 1, 3, 10 or 30 grams per hectare. Adjuvants were added at the standard rate of 0.2% v/v of the spray volume used. The known adjuvants Brij™ 96 and TEHP (tris 2-ethylhexyl phosphate) were also tested for comparison. Each experiment was replicated four times and the results were averaged at each rate. Plants were examined for both protective action and curative effects. A standard mathematical analytical technique was used: Plots of efficacy against pesticide concentration for each adjuvant were 'logit' transformed and used to estimate the concentration required for 90% effect (ED90). For each sample, its ED90 value was compared to that of the known adjuvant Brij™96 or TEHP in order to generate a relative potency; the relative potency is the ratio of the ED90 values. Relative potencies are given in Table 2 [relative potency results compared to Brij™96 for difenoconazole] and Table 3 [relative potency results compared to TEHP for cyproconazole]. Adjuvants which performed better than the standard adjuvants have a relative potency value greater than 1.

**Table 2**

| **Sample** | **Protective** | **Curative** |
|---|---|---|
| 15 | 0.99 | 1.50 |
| 17 | 1.47 | 1.69 |
| 24 | 1.46 | 2.84 |
| 25 | 1.22 | 1.07 |

**Table 3**

| **Sample** | **Protective** | **Curative** |
|---|---|---|
| 15 | 1.80 | 3.81 |
| 17 | 0.97 | 2.73 |
| 24 | 1.68 | 2.97 |
| 25 | 1.22 | 4.29 |

### EXAMPLE 3

In this example Sample 13 from Table 1 was compared to the oil adjuvant blend Turbocharge™. The herbicide fomesafen was applied to the weed species xanthium strumarium (XANST), setaria viridis (SETVI), abutilon theophrasti (ABUTH), and chenopodium album (CHEAL) at rates of 60 or 120 grams per hectare, using a laboratory track sprayer. Each experiment was replicated four times and the percentage damage to the weeds was assessed visually at time periods of 7, 14 and 21 days after application. Each adjuvant was applied at a rate of 0.5% of the volume of the spray water. Four effects were evaluated: pesticide rate (2 levels), adjuvant type (two levels), weed species (four levels) and days after application (three levels). A standard simple linear model was constructed to judge the significance of these effects. These effects were found to be significant at a 5% level. A model was fitted using multiple linear regression using the statistics package JMP (SAS group). The effect of each adjuvant was pulled out from the model and the least significant differences evaluated using a Student's t method. In further examples, where a greater number than two adjuvants were compared, Tukey's HSD method was used.

Table 4 shows the mean efficacy of the butylene oxide adjuvant number 13 with the pesticide fomesafen compared to the oil adjuvant Turbocharge across four weed species and compared to single weed species. The mean values and a letter code denoting significant difference (samples with the same letter are not significantly different from each other at the 5% level.) In addition the mean values split according to individual weed species are also shown. As can be seen, Sample 13 was more efficacious than the standard adjuvant Turbocharge™ across the weed species, and was as good or better on individual weeds.

**Table 4**

| **Sample** | **All weeds** | **XANST** | **CHEAL** | **SETVI** | **ABUTH** |
|---|---|---|---|---|---|
| Sample 13 | 61.8; A | 89.7; A | 89.4; A | 27.5; A | 40.55; A |
| Turbocharge | 58.8; B | 83.9; B | 88.6; A | 24.4; B | 38.33; A |

### EXAMPLE 4

In this example Sample 13 from Table 1 was compared to the adjuvant Brij™96V. The herbicide fomesafen was applied to the weed species xanthium strumarium (XANST), setaria viridis (SETVI), abutilon theophrasti (ABUTH), and chenopodium album (CHEAL) at rates of 60 or 120 grams per hectare by a laboratory track sprayer. Each experiment was replicated four times and the percentage damage to the weeds was assessed visually at time periods of 7, 14 and 21 days after application. Each adjuvant was applied at a rate of 0.2% of the volume of the spray water.

The same statistical methodology that was applied in Example 3 was used here.

Table 5 shows the mean efficacy of the butylene oxide adjuvant number 13 with the pesticide fomesafen compared to the adjuvant Brij 96 across four weed species and compared to single weed species. The mean values and a letter denoting which group each adjuvant belonged to are shown. In addition the mean values split according to individual weed species are also shown. As can be seen the butylene oxide adjuvant was more efficacious than the standard adjuvant across the weed species, and was as good or better on individual weeds.

**Table 5**

| **Sample** | **All weeds** | **ABUTH** | **CHEAL** | **SETVI** | **XANST** |
|---|---|---|---|---|---|
| Sample 13 | 57.15; A | 32.5; A | 85.27; A | 24.17; A | 86.66; A |
| Brij 96V | 53.33; B | 32.2; A | 82.5; A | 26.66; A | 71.94; B |

### EXAMPLE 5

In this example the rate response of the butylene oxide adjuvant number 13 of Table 1 was measured to display the excellent performance of this adjuvant at very low rates. compared to the adjuvant Brij™96V. The herbicide fomesafen was applied to the weed species xanthium strumarium (XANST), setaria viridis (SETVI), abutilon theophrasti (ABUTH), and chenopodium album (CHEAL) at rates of 60 or 120 grams per hectare by a laboratory track sprayer. Each experiment was replicated four times and the percentage damage to the weeds was assessed visually at time periods of 7, 14 and 21 days after application. The adjuvants were each applied at a rate of 0.2% of the volume of the spray water.

The same statistical methodology that was applied in Example 3 was used here. Where a sample has more than one letter it is not significantly different to any other sample with one of those letters.

Table 6 shows the mean efficacy of the butylene oxide adjuvant Sample 13 at several addition rates with the pesticide fomesafen compared to the adjuvant Brij 96V across four weed species, the mean values and a letter denoting which group each adjuvant belonged to are shown. As can be seen the butylene oxide adjuvant was more efficacious than the standard adjuvant across the weed species. In addition it was as effective at half the rate of the standard Brij96V (Sample 13 at 0.1% cf. Brij 96V at 0.2%). The level of addition of the butylene oxide adjuvant that was statistically better than no adjuvant was 0.025%. This is a very low level of adjuvant addition, indicating the remarkable efficacy of this adjuvant.

**Table 6**

| **Sample** | **Adjuvant rate (%)** | **Rank** | **Mean % kill** |
|---|---|---|---|
| Sample 13 | 0.5 | A | 61.805 |
| Sample 13 | 0.2 | B | 57.15 |
| Sample 13 | 0.1 | BC | 54.23 |
| Brij 96 | 0.2 | C | 53.33 |
| Sample 13 | 0.05 | D | 43.95 |
| Sample 13 | 0.025 | E | 38.12 |
| No adjuvant | 0 | F | 27.57 |

### EXAMPLE 6

In this example the butylene oxide adjuvant Sample 13 of Table 1 was compared to the adjuvant Tween™ 20. The herbicide mesotrione was applied at rates of 45 and 90 grams per hectare by a laboratory track sprayer to the weed species brachiaria platyphyla (BRAPL), digitaria sanguinalis (DIGSA), polygonum convolvulus (POLCO) and amaranthus tuberculatus (AMATU). In each case the experiments were replicated four times and the percentage damage to the weeds was assessed visually at time periods of 7, 14 and 21 days after application. The adjuvants was applied at a rate of 0.5% of the volume of the spray water.

The same statistical methodology that was applied in Example 3 was used here.

Table 7 shows the mean efficacy of the butylene oxide adjuvant Sample 13 at a rate of 0.5 % v/v with the pesticide mesotrione compared to the adjuvant Tween 20 across four weed species and compared to single weed species. The mean values and a letter denoting which group each adjuvant belonged to are shown. In addition the mean values split according to individual weed species are also shown. As can be seen the butylene oxide adjuvant was more efficacious than the standard adjuvant across the weed species, and was as good or better on individual weeds.

**Table 7**

| **Sample** | **All weeds** | **BRAPL** | **DIGSA** | **POLCO** | **AMATU** |
|---|---|---|---|---|---|
| Sample 13 | 69.986; A | 54.72; A | 56.94; A | 97.167; A | 71.11; A |
| Tween 20 | 63.861; B | 44.72; B | 48.33; B | 95.44;A | 66.94; AB |
| No adjuvant | 44.986; C | 20.27; C | 19.44; C | 77.16; B | 63.05; B |

### EXAMPLE 7

In this example the butylene oxide adjuvant Sample 13 of Table1 was compared to the adjuvant Brij™96V. The herbicide mesotrione was applied to the weed species brachiaria platyphyla (BRAPL), digitaria sanguinalis (DIGSA), polygonum convolvulus (POLCO) and amaranthus tuberculatus (AMATU) at rates of 45 or 90 grams per hectare by a laboratory track sprayer. Each experiment was replicated four times and the percentage damage to the weeds was assessed visually at time periods of 7, 14 and 21 days after application. The adjuvants were applied at a rate of 0.2% of the volume of the spray water.

The same statistical methodology that was applied in Example 3 was used here.

Table 8 shows the mean efficacy of the butylene oxide adjuvant Sample 13 at a rate of 0.2% v/v with the pesticide mesotrione compared to the adjuvant Brij 96V across four weed species, and compared to single weed species. The mean values and a letter denoting which group each adjuvant belonged to are shown. In addition the mean values split according to individual weed species are also shown. As can be seen the butylene oxide adjuvant was more efficacious than the standard adjuvant across the weed species, and was as good or better on individual weeds.

**Table 8**

| **Sample** | **All weeds** | **BRAPL** | **DIGSA** | **POLCO** | **AMATU** |
|---|---|---|---|---|---|
| Sample 13 | 68.44; A | 51.66; A | 54.72; A | 97.11; A | 70.28; A |
| Brij 96V | 61.4; B | 41.94; B | 44.72; B | 93.66; B | 65.28; B |
| No adjuvant | 44.99; C | 20.27; C | 19.44; C | 77.16; C | 63.05; B |

### EXAMPLE 8

In this example the rate response of the butylene oxide adjuvant Sample 13 of Table 1 was measured to display the excellent performance of this adjuvant at very low rates compared to the adjuvant Brij™96V. The herbicide mesotrione was applied to the weed species brachiaria platyphyla (BRAPL), digitaria sanguinalis (DIGSA), polygonum convolvulus (POLCO) and amaranthus tuberculatus (AMATU) at rates of 45 or 90 grams per hectare by a laboratory track sprayer. Each experiment was replicated four times and the percentage damage to the weeds was assessed visually at time periods of 7, 14 and 21 days after application. The butylene oxide adjuvant was applied at rates of 0.025, 0.05, 0.1, 0.2 and 0.5% w/v of the spray water. For comparison Brij96V was added at a rate of 0.2% w/v.

The same statistical methodology that was applied in Example 3 was used here.

Table 9 shows the mean efficacy of the butylene oxide adjuvant Sample 13 at several addition rates with the pesticide mesotrione compared to the adjuvant Brij 96V across four weed species. The mean values and a letter denoting which group each adjuvant belonged to are shown. As can be seen half the rate of the butylene oxide adjuvant was more efficacious than the standard adjuvant (0.1 % vs 0.2 %). In addition it was as effective at one quarter of the rate of the standard Brij96V (0.05 % vs 0.2%). The level of addition of the butylene oxide adjuvant that was statistically better than no adjuvant was 0.025 %. This is a very low level of adjuvant addition indicating the remarkable efficacy of this adjuvant.

**Table 9**

| **Sample** | **Adjuvant rate %** | **Rank** | **Mean % kill** | **Standard error** |
|---|---|---|---|---|
| Sample 13 | 0.5 | **A** | 70.0 | 1.8737 |
| Sample 13 | 0.2 | **AB** | 68.4 | |
| Sample 13 | 0.1 | **BC** | 66.2 | |
| Sample 13 | 0.05 | **CD** | 63.0 | |
| Brij 96 | 0.2 | **D** | 61.4 | |
| Sample 13 | 0.025 | **D** | 59.5 | |
| No adjuvant | 0 | **E** | 45.0 | |

### EXAMPLE 9

In this example the rate response of the butylene oxide adjuvant Sample 13 of Table 1 was measured to display the excellent performance of this adjuvant at very low rates. The herbicide pinoxaden was applied to the weed species lolium perenne (LOLPE), alopecurius myosuirides (ALOMY), setaria viridis (SETVI) and avena fatua (AVEFA) at rates of 7.5 or 15 grams per hectare by a laboratory track sprayer. Each experiment was replicated four times and the percentage damage to the weeds was assessed visually at time periods of 14 and 21 days after application. The butylene oxide adjuvant was applied at rates of 0.025, 0.05, 0.1, 0.2 and 0.5% w/v of the spray water.

The same statistical methodology that was applied in Example 3 was used here.

Table 10 shows the mean efficacy of the butylene oxide adjuvant Sample 13 at several addition rates with the pesticide pinoxaden. Results are meaned across four weed species. The mean values and a letter denoting which group each adjuvant belonged to are shown. The results show that the butylene oxide adjuvant is efficacious at a very low level (0.025%) and that there is a strong rate response to the added adjuvant.

**Table 10**

| **Sample** | **Adjuvant rate %** | **Rank** | **Mean % kill** | **Standard error** |
|---|---|---|---|---|
| Sample 13 | 0.5 | A | 76.89 | 1.441 |
| Sample 13 | 0.2 | B | 73.89 | |
| Sample 13 | 0.1 | B | 73.45 | |
| Sample 13 | 0.05 | C | 67.53 | |
| Sample 13 | 0.025 | D | 60.76 | |
| No adjuvant | 0 | E | 4.72 | |

### EXAMPLE 10

In this example the butylene oxide adjuvant Sample 13 of Table 1 was compared to the commercial oil adjuvant blend Atplus™411F. The herbicide nicosulfuron was applied to the weed species chenopodium album (CHEAL), digitaria sanguinalis (DIGSA), setaria viridis (SETVI) and abutilon theophrasti (ABUTH) at rates of 30 or 60 grams per hectare by a laboratory track sprayer. Each experiment was replicated four times and the percentage damage to the weeds was assessed visually at time periods of 14 and 21 days after application. The adjuvants were applied at a rate of 0.5% of the volume of the spray water.

The same statistical methodology that was applied in Example 3 was used here.

Table 11 shows the mean efficacy of the butylene oxide adjuvant Sample 13 at a rate of 0.5%v/v with the pesticide nicosulfuron compared to the oil adjuvant Atplus™411F across four weed species and compared to single weed species. The mean values and a letter denoting which group each adjuvant belonged to are shown. In addition the mean values split according to individual weed species are also shown. As can be seen the butylene oxide adjuvant was more efficacious than the standard adjuvant across the weed species, and was as good or better on individual weeds.

**Table 11**

| **Sample** | **All weeds** | **CHEAL** | **SETVI** | **DIGSA** | **ABUTH** |
|---|---|---|---|---|---|
| Sample 13 | 76.15; A | 84.58; A | 90; A | 81.25; A | 48.75; A |
| Atplus 411F | 66.75; B | 74.17; B | 81.58; B | 78.75; A | 32.5; B |
| No adjuvant | 20.94; C | 12.08; C | 41.67; C | 8.75; B | 21.25; C |

### EXAMPLE 11

In this example the butylene oxide adjuvant Sample 13 of Table 1 was compared to the commercial adjuvant tris 2-ethylhexyl phosphate (TEHP). The herbicide nicosulfuron was applied to the weed species chenopodium album (CHEAL), digitaria sanguinalis (DIGSA), setaria viridis (SETVI) and abutilon theophrasti (ABUTH)at rates of 30 or 60 grams per hectare by a laboratory track sprayer. Each experiment was replicated four times and the percentage damage to the weeds was assessed visually at time periods of 14 and 21 days after application. The adjuvants were applied at a rate of 0.2% of the volume of the spray water.

The same statistical methodology that was applied in Example 3 was used here.

Table 12 shows the mean efficacy of the butylene oxide adjuvant Sample 13 at a rate of 0.2% v/v with the pesticide nicosulfuron compared to the adjuvant TEHP the same rate across four weed species, and compared to single weed species. The mean values and a letter denoting which group each adjuvant belonged to are shown along with the standard error. In addition the mean values split according to individual weed species are also shown. As can be seen the butylene oxide adjuvant was more efficacious than the standard adjuvant across the weed species, and was as good or better on individual weeds.

**Table 12**

| **Sample** | **All weeds** | **CHEAL** | **SETVI** | **DIGSA** | **ABUTH** |
|---|---|---|---|---|---|
| Sample 13 | 68.23; A | 76.67; A | 80; A | 73.33; A | 42.92; A |
| TEHP | 58.12; B | 68.33; B | 77.08; A | 69.17; A | 17.92; B |
| No adjuvant | 20.94; C | 12.08; C | 41.67; B | 8.75; B | 21.25; B |

### EXAMPLE 12

In this example the rate response of the butylene oxide adjuvant Sample 13 of Table 1 was measured and compared to the adjuvant TEHP. The herbicide nicosufuron was applied to the weed species chenopodium album (CHEAL), digitaria sanguinalis (DIGSA), setaria viridis (SETVI) and abutilon theophrasti (ABUTH) at rates of 30 to 60 grams per hectare by a laboratory track sprayer. Each experiment was replicated four times and the percentage damage to the weeds was assessed visually at time periods of 14 and 21 days after application. The butylene oxide adjuvant was applied at rates of 0.025, 0.05, 0.1, 0.2 and 0.5% w/v of the spray water. For comparison TEHP was added at a rate of 0.2% w/v.

The same statistical methodology that was applied in Example 3 was used here.

Table 13 shows the mean efficacy of the butylene oxide adjuvant Sample 13 at several addition rates with the pesticide nicosulfuron. Results are meaned across four weed species. As a comparison the adjuvant TEHP was added at 0.2% v/v. The mean values and a letter denoting which group each adjuvant belonged to are shown. As can be seen half the rate of the butylene oxide adjuvant was more efficacious than the standard adjuvant (Sample 13 at 0.1% compared to TEHP at 0.2%). In addition it was as effective at one quarter of the rate of the standard TEHP (0.05% compared to 0.2%). The level of addition of the butylene oxide adjuvant that was statistically better than no adjuvant was 0.025%. This is a very low level of adjuvant addition, indicating the remarkable efficacy of this adjuvant.

**Table 13**

| **Sample** | **Adjuvant rate %** | **Rank** | **Mean % kill** | **Standard error** |
|---|---|---|---|---|
| Sample 13 | 0.5 | A | 76.14 | 1.803 |
| Sample 13 | 0.2 | B | 68.23 | |
| Sample 13 | 0.1 | C | 63.23 | |
| TEHP | 0.2 | D | 58.12 | |
| Sample 13 | 0.05 | D | 56.35 | |
| Sample 13 | 0.025 | E | 51.87 | |
| No adjuvant | 0 | F | 20.93 | |

### EXAMPLE 13

In this example the responses of the butylene oxide adjuvants 13, 17, 19, 21, 22 and 23 of Table 1 were measured at an application rate of 0.2% of the volume of the spray solution used. They were compared to the commercial tank mix adjuvant Atplus™411F, which was applied at the recommended rate of 0.5% by volume. The herbicide nicosufuron was applied to the weed species chenopodium album (CHEAL), digitaria sanguinalis (DIGSA), setaria viridis (SETVI) and abutilon theophrasti (ABUTH) at rates of 30 or 60 grams per hectare by a laboratory track sprayer. Each experiment was replicated four times and the percentage damage to the weeds was assessed visually at time periods of 14 and 21 days after application.

The same statistical methodology that was applied in Example 3 was used here.

Table 14 shows the mean efficacy of six butylene oxide adjuvants at an addition rate of 0.2% with the pesticide nicosulfuron. Results are meaned across two pesticide rates and each sample was replicated four times. As a comparison the adjuvant Atplus™ 411F was added at 0.5% v/v. The mean values and a letter denoting which group each adjuvant belonged to are shown. The data shows that the butylene oxide adjuvants were at least as good as the standard which was used at a higher rate than the butylene oxide adjuvants (0.2% compared to 0.5%).

**Table 14**

| **Sample** | **All weeds** | **ABUTH** | **CHEAL** | **DIGSA** | **SETVI** |
|---|---|---|---|---|---|
| Sample 13 | 78.44; A | 65.42; A | 85; A | 67.5; A | 95.83; A |
| Sample 21 | 76.45; AB | 66.25; A | 85; A | 60; AB | 94.58; AB |
| Sample 22 | 76.35; AB | 61.67; A | 85.83; A | 62.5; AB | 95.42; A |
| Sample 19 | 75.81; AB | 65; A | 84.17; A | 58.33; BC | 95.75; A |
| Sample 23 | 74.16; AB | 62.92; A | 82.08; A | 57.92; BC | 93.75; AB |
| Atplus 411F | 70.73; B | 60.83; A | 74.17; B | 56.67; BC | 91.25; B |
| Sample 17 | 69.37; B | 58.75; A | 76.67; B | 50.83; C | 91.25; B |
| No adjuvant | 37.5; C | 25.83; B | 24.16; C | 18.33; D | 81.67; C |

### EXAMPLE 14

In this example the responses of the adjuvants 13, 17, 19, 21, 22 and 23 were measured at an application rate of 0.2% of the volume of the spray solution used. They were compared to the adjuvant tris 2-ethylhexyl phosphate (TEHP), which was applied at the rate of 0.5% by volume. The herbicide pinoxaden was applied at rates of 7.5 or 15 grams per hectare by a laboratory track sprayer to the weed species lolium perenne (LOLPE), alopecurius myosuirides (ALOMY), setaria viridis (SETVI) and avena fatua (AVEFA). In each case the experiments were replicated four times and the percentage damage to the weeds was assessed visually at time periods of 14 and 21 days after application.

The same statistical methodology that was applied in Example 3 was used here.

Table 15 shows the mean efficacy of six butylene oxide adjuvants at an addition rate of 0.2% with the pesticide pinoxaden. Results are meaned across two pesticide rates and each sample was replicated four times. As a comparison the adjuvant tris 2 ethylhexyl phosphate was added at 0.5% v/v. The mean values and a letter denoting which group each adjuvant belonged to are shown along with the standard error. The standard TEHP was used at a higher rate than the butylene oxide adjuvants (0.2% compared to 0.5%).

**Table 15**

| **Sample** | **All weeds** | **SETVI** | **LOLPE** | **AVEFA** | **ALOMY** |
|---|---|---|---|---|---|
| Sample 23 | 76.98; A | 78.75; ABC | 78.33; AB | 78.33; AB | 72.5; A |
| Sample 13 | 76.98; A | 83.33; A | 77.5; AB | 80.42; A | 66.67; B |
| Sample 19 | 76.25; A | 80; AB | 77.08; AB | 77.08; AB | 70.83; AB |
| TEHP | 75.63; A | 77.5; ABC | 82.5; A | 73.75; AB | 68.75; AB |
| Sample 22 | 73.96; AB | 78.33; ABC | 72.5; BC | 75.83; AB | 69.17; AB |
| Sample 21 | 72.19; AB | 71.25; C | 76.67; AB | 71.66; B | 69.17; AB |
| Sample 17 | 68.02; B | 72.92; BC | 68.75; C | 73.33; AB | 57.08; C |
| No adjuvant | 15.62; C | 17.08; D | 7.08; D | 23.33; C | 15; D |

### EXAMPLE 15

In this example the rate response of the butylene oxide sample 27 of Table 1 was measured, displaying the excellent performance of this adjuvant compared to the commercial adjuvant Turbocharge™, used at a rate of 0.5% by volume. The herbicide fomesafen was applied to the weed species xanthium strumarium (XANST), setaria viridis (SETVI), abutilon theophrasti (ABUTH), and chenopodium album (CHEAL)at rates of 60 or 120 grams per hectare by a laboratory track sprayer. Each experiment was replicated four times and the percentage damage to the weeds was assessed visually at time periods of 7, 14 and 21 days after application. Sample 27 was applied at a rate of 0.2% of the volume of the spray water.

The same statistical methodology that was applied in Example 3 was used here Table 16 shows the mean efficacy of the butylene oxide adjuvant 27 at a rate of 0.2% v/v with the pesticide fomesafen compared to the adjuvant Turbocharge at a rate of 0.5%. The mean values and a letter denoting which group each adjuvant belonged to are shown along with the standard error. As can be seen the butylene oxide adjuvant was as effective as Turbocharge even although it was applied at 0.2% as opposed to 0.5% for the commercial adjuvant.

**Table 16**

| **Sample** | **All weeds** | **XANST** | **SETVI** | **CHEAL** | **ABUTH** |
|---|---|---|---|---|---|
| Sample 27 | 51.01; A | 73.44; A | 33.44; A | 74.3; A | 22.86; B |
| Turbocharge | 52.89; A | 72.33; A | 28.72; B | 75.69; A | 34.81; A |
| No adjuvant | 22.94; B | 31.55; B | 13.78; C | 30.69; B | 15.75; C |

### EXAMPLE 16

In this example the rate response of the butylene oxide adjuvant 27 of Table 1 was measured to display the excellent performance of this adjuvant compared to the commercial adjuvant Tween™20, used at a rate of 0.5% by volume. The herbicide mesotrione was applied to the weed species brachiaria platyphyla (BRAPL), digitaria sanguinalis (DIGSA), polygonum convolvulus (POLCO) and amaranthus tuberculatus (AMATU) at rates of 45 or 90 grams per hectare by a laboratory track sprayer. Each experiment was replicated four times and the percentage damage to the weeds was assessed visually at time periods of 7, 14 and 21 days after application. Sample 27 was applied at a rate of 0.2% of the volume of the spray water.

The same statistical methodology that was applied in Example 3 was used here.

Table 17 shows the mean efficacy of the butylene oxide adjuvant 27 at a rate of 0.2% v/v with the pesticide mesotrione compared to the adjuvant Tween 20 at a rate of 0.5%. The mean values and a letter denoting which group each adjuvant belonged to are shown. As can be seen the butylene oxide adjuvant was as effective as Tween 20 even although it was applied at 0.2% as opposed to 0.5% for the commercial adjuvant.

**Table 17**

| **Sample** | **All weeds** | **POLCO** | **DIGSA** | **BRAPL** | **AMATU** |
|---|---|---|---|---|---|
| Sample 27 | 57.26; A | 75.44; A | 44.99; A | 36.94; A | 71.64; A |
| Tween 20 | 54.54; A | 73.61; A | 41.67; A | 33.06; B | 69.86; A |
| No adjuvant | 34.84; B | 55.5; B | 20.55; B | 19.17; C | 44.13; B |

### EXAMPLE 17

In this example the response of butylene oxide sample 27 of Table 1 was measured at an application rate of 0.2% of the volume of the spray solution used. It was compared to the adjuvant Genopol™X080, which was also applied at a rate of 0.2% by volume. This adjuvant has the same alkyl chain and ethylene oxide head group as Sample 27 however it does not contain the butylene oxide moiety. The herbicide pinoxaden was applied at rates of 7.5 or 15 grams per hectare by a laboratory track sprayer to the weed species lolium perenne (LOLPE), alopecurius myosuirides (ALOMY), setaria viridis (SETVI) and avena fatua (AVEFA). Each experiment was replicated four times and the percentage damage to the weeds was assessed visually at time periods of 14 and 21 days after application.

The same statistical methodology that was applied in Example 3 was used here.

Table 18 shows the mean efficacy of the butylene oxide adjuvant 27 at a rate of 0.2% v/v with the pesticide pinoxaden compared to the adjuvant Genapol X080 at the same rate. The mean values and a letter denoting which group each adjuvant belonged to are shown. The data shows that the butylene oxide adjuvants was significantly more efficacious than Genapol X080 across the range of weed species tested.

**Table 18**

| **Sample** | **All weeds** | **SETVI** | **LOLPE** | **AVEFA** | **ALOMY** |
|---|---|---|---|---|---|
| Sample 27 | 71.06; A | 84.4; A | 59.22; A | 82.3; A | 58.31; A |
| Genopol X080 | 52.08; B | 70.67; B | 20.97; B | 75.47; B | 41.23; B |
| No adjuvant | 7.44; C | 4.75; C | 6.53; C | 5.74; C | 12.73; C |

### EXAMPLE 18

In this example the response of butylene oxide sample 27 of Table 1 was measured at an application rate of 0.2% by volume of the spray solution used. It was compared to the commercial tank mix adjuvant Atplus™411F, which was applied at the recommended rate of 0.5% by volume, and to the adjuvant Genapol™X080 which was applied at 0.2%. This adjuvant has the same alkyl chain and ethylene oxide head group as sample 27 however it does not contain the butylene oxide moiety. The herbicide nicosufuron was applied at rates of 30 or 60 grams per hectare by a laboratory track sprayer to the weed species chenopodium album (CHEAL), digitaria sanguinalis (DIGSA), setaria viridis (SETVI) and abutilon theophrasti (ABUTH). In each case the experiments were replicated four times and the percentage damage to the weeds was assessed visually at time periods of 14 and 21 days after application.

The same statistical methodology that was applied in Example 3 was used here.

Table 19 shows the mean efficacy of the butylene oxide adjuvant 27 at a rate of 0.2 % v/v with the pesticide nicosulfuron compared to the adjuvant Genapol X080 at the same rate, and to Atplus 411F applied at the higher rate of 0.5%. The mean values and a letter denoting which group each adjuvant belonged to are shown. The data shows that the butylene oxide adjuvants was as good as Atplus 411F which was used at a higher rate than the butylene oxide adjuvants (0.2% compared to 0.5%). It was more efficacious than Genapol X080.

**Table 19**

| **Sample** | **All weeds** | **SETVI** | **DIGSA** | **CHEAL** | **ABUTH** |
|---|---|---|---|---|---|
| Sample 27 | 70.26; A | 45.13; A | 79.55; A | 84.58; A | 71.76; A |
| Atplus 411F | 65.74; A | 25.96; B | 76.05; A | 85.01; A | 75.93; A |
| Genopol X080 | 46.47; B | 19.72; C | 42.71; B | 70.42; B | 53.01; B |
| No adjuvant | 21.39; C | 14.17; D | 22.59; C | 15.2; C | 33.6; C |

### EXAMPLE 19

In this example the rate response of the butylene oxide adjuvant 13 of Table 1 was measured to display the excellent performance of this adjuvant compared to the commercial adjuvant Turbocharge™, used at a rate of 0.5% by volume. The herbicide fomesafen was applied at rates of 60, 90 or 120 grams per hectare by a laboratory track sprayer to the weed species xanthium strumarium (XANST), abutilon theophrasti (ABUTH), and chenopodium album (CHEAL). In each case the experiments were replicated four times and the percentage damage to the weeds was assessed visually at time periods of 14 and 21 days after application. Sample 13 was applied at a rate of 0.2% of the volume of the spray water.

The same statistical methodology that was applied in Example 3 was used here.

Table 21 shows the mean efficacy of the butylene oxide adjuvant 13 at a rate of 0.2% v/v with the pesticide fomesafen compared to the adjuvant Turbocharge applied at the higher rate of 0.5%. The mean values and a letter denoting which group each adjuvant belonged to are shown. As can be seen the butylene oxide adjuvant was more effective than Turbocharge even although it was applied at 0.2% as opposed to 0.5% for the commercial adjuvant.

**Table 20**

| **Sample** | **All weeds** | **XANST** | **CHEAL** | **ABUTH** |
|---|---|---|---|---|
| Sample 13 | 51.24; A | 92.56; A | 62.5; A | 41.39; A |
| Turbocharge | 39.43; B | 77.44; B | 40; B | 35; B |
| No adjuvant | 23.61; C | 45; C | 24.44; C | 25; C |

### EXAMPLE 20

In this example the rate response of the butylene oxide adjuvant 13 of Table 1 was measured to display the excellent performance of this adjuvant compared to the commercial adjuvant Tween™20, used at a rate of 0.5% by volume. The herbicide mesotrione was applied at rates of 30, 60 or 90 grams per hectare by a laboratory track sprayer to the weed species brachiaria platyphyla (BRAPL), digitaria sanguinalis (DIGSA), and polygonum convolvulus (POLCO). Sample 13 was applied at a rate of 0.2% of the volume of the spray water. In each case the experiments were replicated four times and the percentage damage to the weeds was assessed visually at time periods of 14 and 21 days after application.

The same statistical methodology that was applied in Example 3 was used here.

Table 21 shows the mean efficacy of the butylene oxide adjuvant 13 at a rate of 0.2% v/v with the pesticide mesotrione compared to the adjuvant Tween 20 applied at the higher rate of 0.5%. The mean values and a letter denoting which group each adjuvant belonged to are shown. As can be seen the butylene oxide adjuvant was more effective than Tween 20 even though it was applied at 0.2% as opposed to 0.5 % for the commercial adjuvant.

**Table 21**

| **Sample** | **All weeds** | **POLCO** | **DIGSA** | **BRAPL** |
|---|---|---|---|---|
| Sample 13 | 28.89; A | 36.11; A | 47.5; A | 39.14; A |
| Tween 20 | 26.11; B | 32.77; B | 45.55; A | 26.11; B |
| No adjuvant | 13.61; C | 27.78; C | 17.5; B | 9.17; C |

### EXAMPLE 21

In this example the response of the butylene oxide adjuvant 13 of Table 1 was measured at an application rate of 0.2% of the volume of the spray solution used. It was compared to the commercial tank mix adjuvant Atplus™411F, which was applied at the recommended rate of 0.5% by volume. The herbicide nicosufuron was applied at rates of 30, 45 or 60 grams per hectare by a laboratory track sprayer to the weed species chenopodium album (CHEAL), digitaria sanguinalis (DIGSA), and abutilon theophrasti (ABUTH). In each case the experiments were replicated four times and the percentage damage to the weeds was assessed visually at time periods of 14 and 21 days after application.

The same statistical methodology that was applied in Example 3 was used here.

Table 22 shows the mean efficacy of the butylene oxide adjuvant 13 at a rate of 0.2% v/v with the pesticide nicosulfuron compared to the adjuvant Atplus 411F applied at the higher rate of 0.5%. The mean values and a letter denoting which group each adjuvant belonged to are shown. The data shows that the butylene oxide adjuvant was as good as Atplus 411F which was used at a higher rate than the butylene oxide adjuvant (0.5% compared to 0.2%).

**Table 22**

| **Sample** | **All weeds** | **DIGSA** | **CHEAL** | **ABUTH** |
|---|---|---|---|---|
| Sample 13 | 67.31; A | 78.61; A | 88.33; A | 35; A |
| Atplus 411F | 63.61; A | 75.55; B | 88.06; A | 27.22; B |
| No adjuvant | 8.11; B | 4.05; C | 0.56; B | 19.72; C |

### EXAMPLE 22

In this example the response of the butylene oxide adjuvant 13 of Table 1 was measured at an application rate of 0.2% of the volume of the spray solution used. It was compared to the adjuvant tris 2-ethylhexyl phosphate [TEHP], which was applied at the higher rate of 0.5% by volume. The herbicide pinoxaden was applied at rates of 7.5, 11.25 or 15 grams per hectare by a laboratory track sprayer to the weed species lolium perenne (LOLPE), alopecurius myosuirides (ALOMY), and avena fatua (AVEFA). In each case the experiments were replicated four times and the percentage damage to the weeds was assessed visually at time periods of 14 and 21 days after application.

The same statistical methodology that was applied in Example 3 was used here.

Table 23 shows the mean efficacy of butylene oxide adjuvant 13 at a rate of 0.2% v/v with the pesticide pinoxaden compared to the adjuvant tris 2-ethylhexyl phosphate applied at the higher rate of 0.5%. The mean values and a letter denoting which group each adjuvant belonged to are shown. The data shows that the butylene oxide adjuvant was as effective as TEHP across the range of weed species tested.

**Table 23**

| **Sample** | **All weeds** | **LOLPE** | **AVEFA** | **ALOMY** |
|---|---|---|---|---|
| Sample 13 | 58.05; A | 53.89; B | 76.94; A | 43.33; A |
| TEHP | 59.91; A | 58.06; A | 78.33; A | 43.33; A |
| No adjuvant | 3.43; B | 2.22; C | 6.39; B | 1.67; B |

### EXAMPLE 23

In this example the rate response of 22 butylene oxide adjuvants of Table 1 were measured at an adjuvant rate of 0.2% by volume, displaying excellent performance of this adjuvant compared to the commercial adjuvant Turbocharge, which was used at a rate of 0.5% by volume. The herbicide mesotrione was applied at rates of 60 or 120 grams per hectare by a laboratory track sprayer to the weed species brachiaria platyphyla (BRAPP), digitaria sanguinalis (DIGSA), abutilon theophrasti (ABUTH) and amaranthus retroflexus (AMARE). In each case the experiments were replicated four times and the percentage damage to the weeds was assessed visually at time periods of 14 and 21 days after application. All the butylene oxide adjuvants were applied at a rate of 0.2% volume of the spray water whereas Turbocharge was used at 0.5%.

The same statistical methodology that was applied in Example 3 was used here.

Table 23 shows the mean efficacy of 22 butylene oxide adjuvants used at a rate of 0.2% v/v with the pesticide mesotrione compared to the adjuvant Turbocharge applied at the higher rate of 0.5%. The mean values and a letter denoting which group each adjuvant belonged to are shown. As can be seen most of the butylene oxide adjuvants were more effective than Turbocharge even although they were applied at 0.2% as opposed to 0.5% for the commercial adjuvant. All of the butylene adjuvants were as good as Turbocharge.

**Table 23**

| **Sample** | **Group** | **Mean kill across weeds %** |
|---|---|---|
| 3 | A | 84.69 |
| 27 | AB | 84.27 |
| 26 | ABC | 83.85 |
| 21 | ABCD | 83.65 |
| 13 | ABCDE | 83.44 |
| 20 | ABCDE | 83.44 |
| 23 | ABCDE | 83.44 |
| 19 | ABCDEF | 83.13 |
| 7 | ABCDEF | 83.02 |
| 2 | ABCDEF | 82.60 |
| 6 | ABCDEF | 82.60 |
| 1 | BCDEFG | 81.98 |
| 22 | CDEFGH | 81.88 |
| 18 | CDEFGH | 81.77 |
| 25 | CDEFGH | 81.67 |
| 10 | DEFGHI | 81.35 |
| 17 | EFGHI | 81.25 |
| 11 | FGHI | 80.94 |
| 8 | GHIJ | 80.21 |
| 24 | HIJ | 79.58 |
| 16 | IJ | 79.27 |
| Turbocharge | J | 78.44 |
| 5 | J | 78.13 |
| No adjuvant | K | 69.48 |

### EXAMPLE 24

In this example the responses of the butylene oxide adjuvants 7, 14, 24 and 25 of Table 1 were measured at an application rate of 0.2% of the volume of the spray solution used. They were compared to the adjuvant Brij™96V, which was applied at the same rate. The herbicide pinoxaden was applied to the weed species lolium perenne (LOLPE), alopecurius myosuirides (ALOMY), setaria viridis (SETVI) and avena fatua (AVEFA) at rates of 7.5 or 15 grams per hectare by a laboratory track sprayer. Each experiment was replicated three times and the percentage damage to the weeds was assessed visually 13 days after application.

The same statistical methodology that was applied in Example 3 was used here.

Table 25 shows the mean efficacy of 4 butylene oxide adjuvants used at a rate of 0.2% v/v with the pesticide pinoxaden compared to the adjuvant Brij 96V applied at the same rate. The mean values and a letter denoting which group each adjuvant belonged to are shown. The data shows that two of the butylene oxide adjuvants were more effective than Brij 96V and two were as good as Brij 96V across the range of weed species tested.

**Table 25**

| **Sample** | **Group** | **Mean kill % all weeds** |
|---|---|---|
| 7 | A | 72.3 |
| 14 | A | 67.3 |
| 25 | B | 57.3 |
| Brij 96V | B | 55.0 |
| 24 | B | 52.9 |
| No adjuvant | C | 16.3 |

### EXAMPLE 25

In this example the rate responses of four butylene oxide adjuvants ofTable 1, applied at 0.2%, displayed excellent performance when compared to the commercial adjuvant Turbocharge™, used at a rate of 0.5%; and to tris 2-ethylhexyl phosphate (TEHP) also used at 0.5%. The herbicide fomesafen was applied to the weed species xanthium strumarium (XANST), abutilon theophrasti (ABUTH), setaria viridis (SETVI) and chenopodium album (CHEAL) at rates of 60 or 120 grams per hectare by a laboratory track sprayer. Each experiment was replicated six times and the percentage damage to the weeds was assessed visually at time periods of 7 and 13 days after application.

The same statistical methodology that was applied in Example 3 was used here.

Table 26 shows the mean efficacy of 4 butylene oxide adjuvants used at a rate of 0.2% v/v with the pesticide fomesafen compared to the adjuvants tris 2-ethylhexyl phosphate (TEHP) and Turbocharge. The latter two adjuvants were applied at the higher rate of 0.5% v/v. The mean values and a letter denoting which group each adjuvant belonged to are shown along with the standard error. As can be seen the performance of all four of the butylene oxide adjuvants was at least as good as the standard Turbocharge and most of the adjuvants were as good as or better than TEHP.

**Table 26**

| **Sample** | **Group** | **Mean kill across weeds (%)** |
|---|---|---|
| 24 | A | 78.2 |
| 7 | B | 73.6 |
| 25 | B | 73.1 |
| TEHP | B | 72.7 |
| Turbocharge | C | 68.7 |
| 14 | C | 66.1 |
| None | D | 47.0 |

## Claims

1. An agrochemical pesticidal formulation comprising a bioperformance enhancing adjuvant compound of formula (I)
R₁O[BO]ₙ[AO]ₘR₂ (I)
where BO is CH(R₄)CH(R₅)O and, independently for each BO unit, R₄ is methyl and R₅ is methyl; or R₄ is ethyl and R₅ is hydrogen; or R₄ is hydrogen and R₅ is ethyl; each AO is independently propylene oxide or ethylene oxide; n is from 1 to 12; m is from 5 to 15; R₁ is C₁₀₋₁₆ alkyl optionally substituted by hydroxy or epoxy groups; and R₂ is hydrogen or C₁₋₃ alkyl; provided that the compound of formula (I) is not 2-propylheptanol+3BO+12EO, 2-propylheptanol+7BO+12EO, 2-propylheptanol+9BO+12EO or 2-propylheptanol+12BO+9EO.

2. An agrochemical pesticidal formulation as claimed in claim 1 where R₂ is hydrogen or C₁₋₂ alkyl.

3. An agrochemical pesticidal formulation as claimed in claim 1 or 2 where the compound of formula (I) is a compound of formula (Ib):
R₁O[BO]ₙ[PO]_{m'}[EO]_{m''}R₂ (Ib)
where (m' + m" = m).

4. An agrochemical pesticidal formulation as claimed in any one of claims 1 to 3 where n is from 2 to 8.

5. An agrochemical pesticidal formulation as claimed in any one of claims 1 to 4 where m is from 8 to 12.

6. An agrochemical pesticidal formulation as claimed in claim 1 where AO is ethylene oxide.

7. Use of a compound of formula (I) as defined in any of the preceding claims to enhance the bioperformance of a pesticide.

## Patentansprüche

1. Agrarchemische pestizide Formulierung, umfassend eine die biologische Leistung fördernde Adjuvansverbindung der Formel (I),
R₁O[BO]ₙ[AO]ₘR₂ (I)
in der BO CH(R₄)CH(R₅)O bedeutet und unabhängig für jede BO-Einheit R₄ Methyl bedeutet und R₅ Methyl bedeutet; oder R₄ Ethyl bedeutet und R₅ Wasserstoff bedeutet; oder R₄ Wasserstoff bedeutet und R₅ Ethyl bedeutet; jedes AO unabhängig Propylenoxid oder Ethylenoxid bedeutet; n 1 bis 12 beträgt; m 5 bis 15 beträgt; R₁ C₁₀₋₁₆-Alkyl, das gegebenenfalls durch Hydroxy- oder Epoxygruppen substituiert ist, bedeutet; und R₂ Wasserstoff oder C₁₋₃-Alkyl bedeutet; mit der Maßgabe, dass die Verbindung der Formel (I) nicht 2-Propylheptanol+3BO+12EO, 2-Propylheptanol+7BO+12EO, 2-Propylheptanol+9BO+12EO oder 2-Propylheptanol+12BO+9EO bedeutet.

2. Agrarchemische pestizide Formulierung nach Anspruch 1, in der R₂ Wasserstoff oder C₁₋₂-Alkyl bedeutet.

3. Agrarchemische pestizide Formulierung nach Anspruch 1 oder 2, wobei es sich bei der Verbindung der Formel (I) um eine Verbindung der Formel (Ib) handelt:
R₁O[BO]ₙ[PO]_{m'}[EO]_{m''}R₂ (Ib)
in der (m' + m'' = m).

4. Agrarchemische pestizide Formulierung nach einem der Ansprüche 1 bis 3, in der n 2 bis 8 beträgt.

5. Agrarchemische pestizide Formulierung nach einem der Ansprüche 1 bis 4, in der m 8 bis 12 beträgt.

6. Agrarchemische pestizide Formulierung nach Anspruch 1, in der AO Ethylenoxid bedeutet.

7. Verwendung einer Verbindung der Formel (I) wie in einem der vorhergehenden Ansprüche definiert zum Fördern der biologischen Leistung eines Pestizids.

## Revendications

1. Formulation pesticide agrochimique, comprenant un composé adjuvant améliorant les performances biologiques, de formule (I)
R₁O[BO]ₙ[AO]ₘR₂ (I)
où BO est CH(R₄)CH(R₅)O et, indépendamment pour chaque motif de BO, R₄ est méthyle et R₅ est méthyle ; ou R₄ est éthyle et R₅ est hydrogène ; ou R₄ est hydrogène et R₅ est éthyle ; chaque AO est indépendamment l'oxyde de propylène ou l'oxyde d'éthylène ; n va de 1 à 12 ; m va de 5 à 15 ; R₁ est C₁₀₋₁₆-alkyle éventuellement substitué par des groupements hydroxy ou époxy ; et R₂ est hydrogène ou C₁₋₃-alkyle ; à condition que le composé de formule (I) ne soit pas 2-propylheptanol + 3BO + 12OE, 2-propylheptanol + 7BO + 12OE, 2-propylheptanol + 9BO + 12OE ou 2-propylheptanol + 12BO + 90E.

2. Formulation pesticide agrochimique selon la revendication 1, dans laquelle R₂ est hydrogène ou C₁₋₂-alkyle.

3. Formulation pesticide agrochimique selon la revendication 1 ou 2, dans laquelle le composé de formule (I) est un composé de formule (Ib) :
R₁O[BO]ₙ[PO]_{m'}[OE]_{m''}R₂ (Ib)
où (m' + m'' = m).

4. Formulation pesticide agrochimique selon l'une quelconque des revendications 1 à 3, dans laquelle n va de 2 à 8.

5. Formulation pesticide agrochimique selon l'une quelconque des revendications 1 à 4, dans laquelle m va de 8 à 12.

6. Formulation pesticide agrochimique selon la revendication 1, dans laquelle AO est l'oxyde d'éthylène.

7. Utilisation d'un composé de formule (I) tel que défini selon l'une quelconque des revendications précédentes, afin d'améliorer les performances biologiques d'un pesticide.
